# EUROPÄISCHE PATENTSCHRIFT

(11) **EP 1 489 064 B1**
(45) Veröffentlichungstag und Bekanntmachung des Hinweises auf die Patenterteilung: **16.04.2008**
(21) Anmeldenummer: 04013211.0
(22) Anmeldetag: 04.06.2004
(51) Int. Cl.: C07C 45/36, C07C 49/80, C07C 49/84

(54) **Herstellung von fluorhaltigen Acetophenonen**
Preparation of fluorinated acetophenones
Préparation d' acétophénones fluorées

(30) Priorität: 16.06.2003 DE 10326917
(43) Veröffentlichungstag der Anmeldung: 22.12.2004
(73) Patentinhaber: Saltigo GmbH, 40764 Langenfeld (DE)
(72) Erfinder: Marhold, Albrecht, Dr., 51373 Leverkusen (DE); Joschek, Jens Peter, Dr., 51061 Köln (DE)
(74) Vertreter: Wichmann, Birgid

(56) Entgegenhaltungen:
- EP-A- 0 460 575
- EP-A- 0 878 234
- ISHII, YASUTAKA ET AL: "Alkane Oxidation with Molecular Oxygen Using a New Efficient Catalytic System : N-Hydroxyphthalimide (NHPI) Combined with Co(acac)n (n = 2 or 3)" JOURNAL OF ORGANIC CHEMISTRY , 61(14), 4520-4526 CODEN: JOCEAH; ISSN: 0022-3263, 1996, XP000591064
- ISHII, YASUTAKA ET AL: "Novel Catalysis by N-Hydroxyphthalimide in the Oxidation of Organic Substrates by Molecular Oxygen" JOURNAL OF ORGANIC CHEMISTRY , 60(13), 3934-5 CODEN: JOCEAH; ISSN: 0022-3263, 1995, XP000512054

## Beschreibung

Die Erfindung betrifft ein Verfahren zur Herstellung von fluorhaltigen Acetophenonen aus fluorhaltigen Arylverbindungen in Gegenwart von N-Hydroxyphthalimiden.

Fluorhaltige Acetophenone wie insbesondere 3-Trifluormethylacetophenon sind wertvolle Zwischenverbindungen für die Synthese von Agro- oder Pharmawirkstoffen (siehe auch EP-A 460 575).

Die Herstellung von acylierten Aromaten erfolgt üblicherweise durch direkte Acylierung von Aromaten in Gegenwart von Lewissäuren, kann aber beispielsweise auch durch Oxidation von alpha-Hydroxyalkylaromaten oder Isoalkylaromaten mit N-Hydroxyphthalimid erfolgen (siehe auch EP-A 796 835; Y. Ishii et al., Catalysis Surveys from Japan, 1999, 3, 27-35; Y. Ishii et al., Adv. Synth. Cat., 2001, 5, 393-427). Nachteilig an den Verfahren ist, dass sie auf elektronenreiche Aromaten beschränkt sind und letztere Methode üblicherweise wenig selektiv verläuft. Für fluorhaltige und andere elektronenarme Aromaten erfolgt die Synthese in der Regel über die Schritte Nitrierung, Reduktion des Nitroaromaten zum entsprechenden Anilin und Diazotierung mit Aldoxim zum entsprechenden Acetophenon. Nachteilig an diesem Verfahren ist, dass es über mehrere Stufen verläuft und somit hohen apparativen Aufwand erfordert und weiterhin die Gesamtausbeute nur mäßig ist.

Es bestand daher das Bedürfnis, ausgehend von einfach verfügbaren Edukten ein effizientes Verfahren zur Herstellung von fluorhaltigen Acetophenonen bereitzustellen.

Überraschend wurde nun ein Verfahren zur Herstellung von Verbindungen der Formel (I) gefunden, 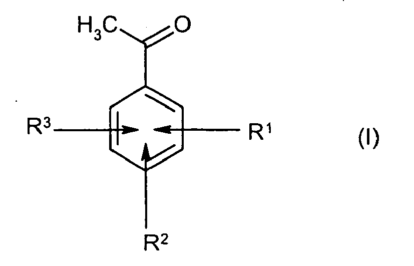 in der
- R¹: für C₁-C₁₂-Perfluoralkyl oder C₁-C₁₂-Perfluoralkoxy
- R²: für Wasserstoff, Fluor, Chlor, Brom, C₁-C₄-Alkyl, C₁-C₄-Alkoxy, Nitro, Cyano oder COOR⁴ steht, wobei R⁴ für Wasserstoff oder C₁-C₄-Alkyl steht und
- R³: für Wasserstoff, Fluor oder Chlor steht, oder
zwei der Reste R¹, R² und R³ zusammen für Difluormethylendioxy, Tetrafluorethylen-1,2-dioxy, 2-Oxy-tetrafluorethyl oder (Oxydifluormethoxy)difluormethyl stehen,
das dadurch gekennzeichnet ist, dass Verbindungen der Formel (IIa) oder (IIb) 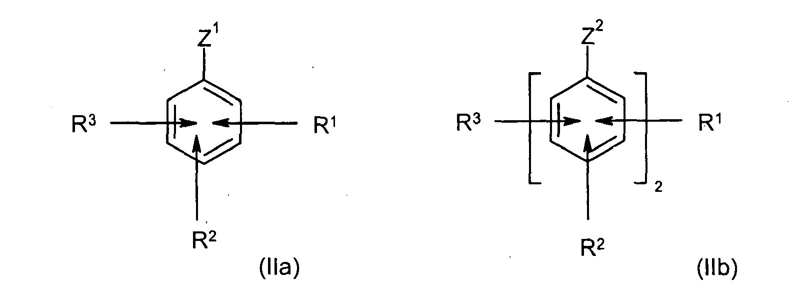 in der
- R¹, R² und R³: die vorstehend genannte Bedeutung besitzen und
- Z': für Isopropyl, Propenyl, 2-Hydroxy-2-propyl, 2-Hydroperoxy-2-propyl oder 1-Methyloxiranyl und
- Z²: für O,O-Bis-(2-oxydiisopropyl) steht

- in Gegenwart von 0,1 bis 30 mol-% eines N-Hydroxyphthalimids
- in Gegenwart eines Oxidationsmittels
umgesetzt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.

Alkyl beziehungsweise Alkoxy steht jeweils unabhängig für einen geradkettigen, cyclischen, verzweigten oder unverzweigten Alkyl- beziehungsweise Alkoxy-Rest. Gleiches gilt für den nichtaromatischen Teil eines Arylalkyl-Restes.

C₁-C₄-Alkyl steht beispielsweise für Methyl, Ethyl, iso-Propyl, n-Butyl, sec.-Butyl und tert.-Butyl.

C₁-C₄-Alkoxy steht beispielsweise für Methoxy, Ethoxy, n-Propoxy, iso-Propoxy, n-Butoxy, sec.-Butoxy und tert.-Butoxy.

**Perfluoralkyl** bzw. **Perfluoralkoxy** steht jeweils für einen geradkettigen, cyclischen, verzweigten oder unverzweigten Alkyl-, bzw. Alkoxy-Rest, der vollständig durch Fluoratome substituiert ist.

Beispielsweise steht C₁-C₁₂-Fluoralkyl für Trifluormethyl, Pentafluorethyl, Heptafluorisopropyl, Nonafluorbutyl, Perfluoroctyl und Perfluordodecyl.

Beispielsweise steht C₁-C₁₂-Fluoralkoxy für Trifluormethoxy, Pentafluorethoxy, Heptafluorisopropoxy und Nonafluorbutoxy.

Im Folgenden werden die bevorzugten Substitutionsmuster für Verbindungen der Formeln (I) und (IIa,b) definiert:
- R¹: steht bevorzugt für C₁-C₄-Perfluoralkyl oder C₁-C₄-Perfluoralkoxy, besonders bevorzugt für Trifluormethyl oder Trifluormethoxy.
- R²: steht bevorzugt für Wasserstoff, Fluor, Chlor, Methyl, Ethyl, Methoxy, Nitro, Cyano oder COOH, besonders bevorzugt für Wasserstoff, Fluor oder Chlor, ganz besonders bevorzugt für Wasserstoff oder Fluor.
- R³: steht bevorzugt für Wasserstoff oder Fluor, besonders bevorzugt für Wasserstoff.

Weiterhin sind Verbindungen bevorzugt, in denen zwei der Reste R¹, R² und R³ zusammen für Difluormethylendioxy oder Tetrafluorethylen-1,2-dioxy stehen.

Bevorzugte Verbindungen sind weiterhin solche, die der Formel (IIa) gehorchen und in denen Z' für Isopropyl und Propenyl steht, wobei Isopropyl noch weiter bevorzugt ist.

Als besonders bevorzugte Einzelverbindungen der Formel (IIa) seien genannt:

3-Trifluormethyl-isopropylbenzol, 4-Trifluormethyl-isopropylbenzol, 5-Isopropyl-2,2-difluorbenzodioxol, 4-Chlor-3-trifluormethyl-isopropylbenzol, 4-Chlor-2-trifluormethyl-isopropylbenzol,4-Trifluormethoxy-isopropylbenzol.

Besonders bevorzugte Verbindungen der Formel (I) sind:

3-Trifluormethyl-isopropylbenzol, 4-Trifluormethyl-isopropylbenzol und 5-Isopropyl-2,2-difluorbenzodioxol.

Werden als Verbindungen der Formel (IIa) solche eingesetzt, in denen Z' für Isopropyl steht werden diese vorzugsweise hergestellt durch Umsetzung von Verbindungen der Formel (III) 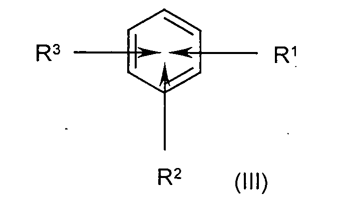 in der
- R¹, R² und R³: die vorstehend genannte Bedeutung besitzen mit Propen oder Isopropylchlorid, vorzugsweise mit Propen, in Gegenwart von Fluorwasserstoff, vorzugsweise in Fluorwasserstoff.

Bei dieser Reaktionsführung können verschiedene Isomere auftreten, die destillativ getrennt werden können und wobei das Fehlisomer vorteilhafterweise zur Reisomerisierung in die Reaktion zurückgeführt werden kann.

Bevorzugte N-Hydroxyphthalimide sind solche der Formeln (IVa) und (IVb) 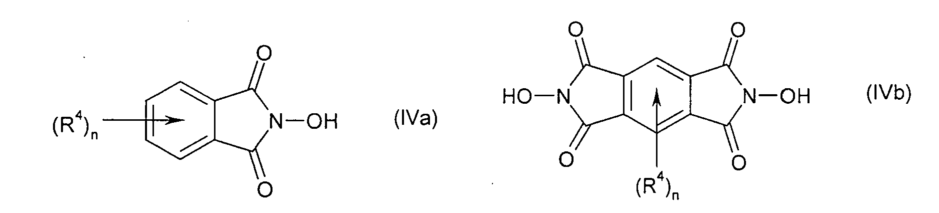 in denen die Reste R⁴ jeweils unabhängig voneinander für Fluor, Chlor, Brom, Trifluormethyl, Cyano oder Nitro stehen, und
- n: für null, eins, zwei, drei oder vier steht.

Besonders bevorzugt sind N-Hydroxyphthalimide der Formel (IVa), ganz besonders bevorzugt ist N-Hydroxyphthalimid.

Gegebenenfalls kann die erfindungsgemäße Umsetzung von Verbindungen der Formeln (IIa) und (IIb) zu Verbindungen der Formel (I) weiterhin
- in Gegenwart von Übergangsmetallen oder Übergangsmetallverbindungen
durchgeführt werden.

Geeignete Übergangsmetalle oder Übergangsmetallverbindungen sind beispielsweise Kobalt, Mangan, Eisen, Kupfer, Rhodium, Osmium, Ruthenium oder deren Verbindungen.

Bevorzugte Übergangsmetalle sind Kobalt, Mangan, Rhodium und Ruthenium als Co-Katalysator für N-Hydroxyphthalimide.

Gegebenenfalls kann die erfindungsgemäße Umsetzung von Verbindungen der Formeln (IIa) und (IIb) zu Verbindungen der Formel (I) weiterhin
- in Gegenwart eines organischen Lösungsmittels
durchgeführt werden.

Bevorzugt sind solche organischen Lösungsmittel die gegen Sauerstoff unter den Reaktionsbedingungen zumindest weitestgehend inert sind und sowohl das Substrat als auch die N-Hydroxyphthalimide bei Raumtemperatur zumindest merklich lösen können. Merklich heißt dabei, dass bei Reaktionstemperatur Lösungen erhalten werden, die mindestens 0,1 molar, vorzugsweise mindestens 0,5 molar an der jeweiligen Komponente sind.

Solche Lösungsmittel sind beispielsweise: Acetonitril, Benzonitril, Polyglycol oder fluorierte Lösungsmittel wie z.B. N(C₂F₅)₃, 3,5-Bis(trifluormethyl)trifluormethoxybenzol oder teil- oder perfluorierte Alkyltetrahydrofurane.

Als Oxidationsmittel können Sauerstoff enthaltende Gase wie insbesondere Sauerstoff selbst, Luft, N₂O oder deren Mischungen eingesetzt werden, wobei Sauerstoff bevorzugt ist.

Der Reaktionsdruck kann beispielsweise bei 0,2 bis 100 bar betragen, bevorzugt 1 bis 20 bar.

Die Reaktionstemperatur kann zum Beispiel 20°C bis 160°C, bevorzugt 40 bis 120°C betragen.

Die Reaktionsdauer kann beispielsweise 1 bis 72 Stunden betragen, bevorzugt 2 bis 24 Stunden.

Die Aufarbeitung der Reaktionsmischungen kann in an sich bekannter Weise beispielsweise durch Destillation erfolgen.

In einer bevorzugten Ausführungsform des erfindungsgemäßen Verfahrens werden nicht oder teilumgesetzte Edukte ebenso wie gegebenenfalls vorhandene Übergangsmetalle oder Übergangsmetallverbindungen und/oder gegebenenfalls vorhandenes organisches Lösungsmittel wieder in die Reaktion zurückgeführt.

Die erfindungsgemäß erhältlichen Verbindungen der Formel (I) eignen sich insbesondere als Zwischenprodukte in einem Verfahren zur Herstellung von Agro- oder Pharmawirkstoffen.

Auf erfindungsgemäße Weise werden die Verbindungen der Formel (I) ausgehend von einfach verfügbaren oder herstellbaren Verbindungen in guten Ausbeuten erhalten, wobei durch die Rückführung in einzelnen Schritten ökonomisch und ökologisch noch vorteilhaftere Verfahrensführungen erreichbar sind.

### Beispiele:

### Beispiel 1

### Darstellung von 3-Trifluormethylacetophenon aus 3-Trifluormethylisopropylbenzol

In einem 500 ml Kolben werden 2,57 g N-Hydroxyphtalimid zusammen mit 15,0 g 3-Isopropylbenzotrifluorid in Benzonitril gelöst und die Reaktionsmischung anschließend entgast. Die Lösung wird mit Sauerstoff gesättigt und auf 100°C erhitzt. Bei dieser Temperatur wird 20 Stunden gerührt. Zur Aufarbeitung wird der Ansatz auf Eis gegeben, über einen Faltenfilter filtriert und mit MTBE nachwaschen. Das Filtrat wird mit MTBE extrahiert, die vereinigten organischen Phasen mit verd. NaOH gewaschen. getrocknet, einrotiert. (MTBE = Methyl-tertiär-butylether) Ausbeute/Umsatz laut GC: 65%d. Th.

## Patentansprüche

1. Verfahren zur Herstellung von Verbindungen der Formel (I), 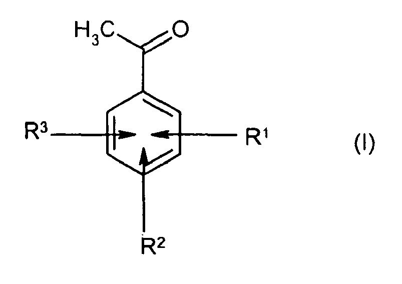 in der
R¹ für C₁-C₁₂-Perfluoralkyl oder C₁-C₁₂-Perfluoralkoxy
R² für Wasserstoff, Fluor, Chlor, Brom, C₁-C₄-Alkyl, C₁-C₄-Alkoxy, Nitro, Cyano oder COOR⁴ steht, wobei R⁴ für Wasserstoff oder C₁-C₄-Alkyl steht und
R³ für Wasserstoff, Fluor oder Chlor steht, oder
zwei der Reste R¹, R² und R³ zusammen für Difluormethylendioxy, Tetrafluorethylen-1,2-dioxy, 2-Oxy-tetrafluorethyl oder (Oxydifluormethoxy)-difluormethyl stehen,
**dadurch gekennzeichnet, dass** Verbindungen der Formel (IIa) oder (IIb) 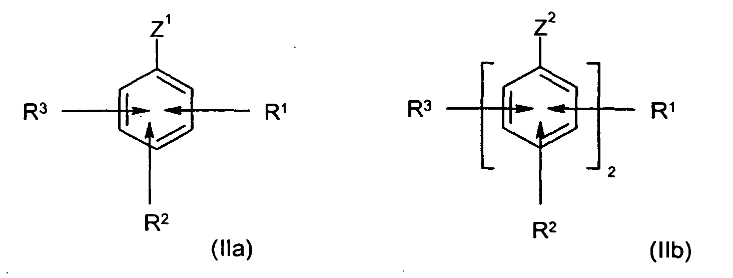 in der
R¹, R² und R³ die vorstehend genannte Bedeutung besitzen und
Z¹ für Isopropyl, Propenyl, 2-Hydroxy-2-propyl, 2-Hydroperoxy-2-propyl oder 1-Methyloxiranyl und
Z² für O,O-Bis-(2-oxydiisopropyl) steht
• in Gegenwart von 0,1 bis 30 mol-% eines N-Hydroxyphthalimids
• in Gegenwart eines Oxidationsmittels
umgesetzt werden.

2. Verfahren nach Anspruch 1, **dadurch gekennzeichnet, dass** Verbindungen der Formel (IIa) eingesetzt werden, in denen Z¹ für Isopropyl und Propenyl steht.

3. Verfahren nach mindestens einem der Ansprüche 1 und 2, **dadurch gekennzeichnet, dass** Verbindungen der Formel (IIa) eingesetzt werden, in denen Z¹ für Isopropyl steht.

4. Verfahren nach Anspruch 3, **dadurch gekennzeichnet, dass** die eingesetzten Verbindungen durch Umsetzung von Verbindungen der Formel (III) 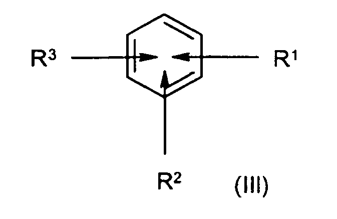 in der
R¹, R² und R³ die in Anspruch 1 genannte Bedeutung besitzen, mit Propen oder Isopropylchlorid in Gegenwart von Fluorwasserstoff erhalten werden.

5. Verfahren nach mindestens einem der Ansprüche 1 bis 4, **dadurch gekennzeichnet, dass** Umsetzung von Verbindungen der Formeln (IIa) und (IIb) zu Verbindungen der Formel (I) weiterhin
• in Gegenwart von Übergangsmetallen oder Übergangsmetallverbindungen
durchgeführt wird.

6. Verfahren nach mindestens einem der Ansprüche 1 bis 5, **dadurch gekennzeichnet, dass** als Oxidationsmittel Sauerstoff enthaltende Gase eingesetzt werden.

7. Verfahren nach mindestens einem der Ansprüche 1 bis 5, **dadurch gekennzeichnet, dass** nicht oder teilumgesetzte Edukte ebenso wie gegebenenfalls vorhandene Übergangsmetalle oder Übergangs-metallverbindungen wieder in die Reaktion zurückgeführt werden.

## Claims

1. Process for preparing compounds of the formula (I) 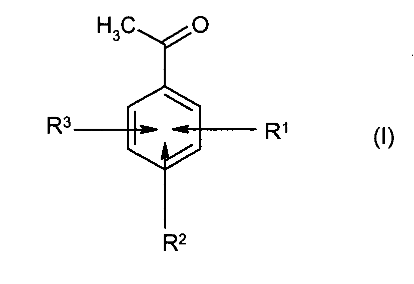 in which
R¹ is C₁-C₁₂-perfluoroalkyl or C₁-C₁₂-perfluoroalkoxy
R² is hydrogen, fluorine, chlorine, bromine, C₁-C₄-alkyl, C₁-C₄-alkoxy, nitro, cyano or COOR⁴ where R⁴ is hydrogen or C₁-C₄-alkyl and
R³ is hydrogen, fluorine or chlorine, or
two of the R¹, R² and R³ radicals together are difluoromethylenedioxy, tetrafluoroethylene-1,2-dioxy, 2-oxytetrafluoroethyl or (oxydifluoromethoxy)difluoromethyl,
**characterized in that** compounds of the formula (IIa) or (IIb) 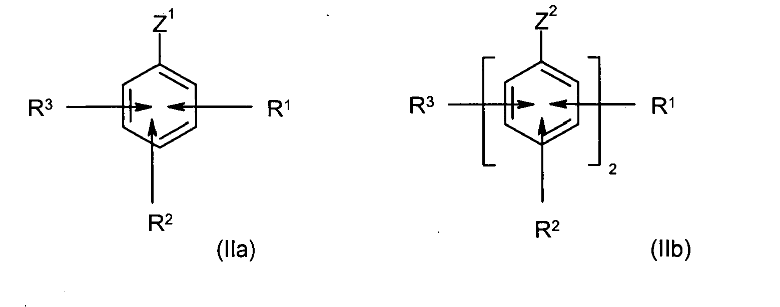 in which
R¹, R² and R³ are each as defined above and
Z¹ is isopropyl, propenyl, 2-hydroxy-2-propyl, 2-hydroperoxy-2-propyl or 1-methyloxiranyl and
Z² is O,O-bis(2-oxydiisopropyl)
are converted
• in the presence of 0.1 to 30 mol% of an N-hydroxyphthalimide
• in the presence of an oxidizing agent.

2. Process according to Claim 1, **characterized in that** compounds of the formula (IIa) are used in which Z¹ is isopropyl and propenyl.

3. Process according to at least one of Claims 1 and 2, **characterized in that** compounds of the formula (IIa) are used in which Z¹ is isopropyl.

4. Process according to Claim 3, **characterized in that** the compounds used are obtained by reacting compounds of the formula (III) 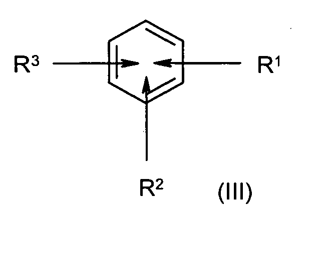 in which
R¹, R² and R³ are each as defined in Claim 1 with propene or isopropyl chloride in the presence of hydrogen fluoride.

5. Process according to at least one of Claims 1 to 4, **characterized in that** conversion of compounds of the formulae (IIa) and (IIb) to compounds of the formula (I) is also carried out
• in the presence of transition metals or transition metal compounds.

6. Process according to at least one of Claims 1 to 5, **characterized in that** the oxidizing agent used is an oxygen-containing gas.

7. Process according to at least one of Claims 1 to 5, **characterized in that** unconverted or part-converted reactants, just like any transition metals or transition metal compounds present, are recycled back into the reaction.

## Revendications

1. Procédé de préparation de composés de formule (I) 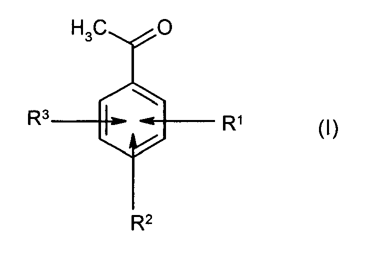 dans laquelle :
R¹ représente un groupe perfluoroalkyle en C₁-C 12 ou un groupe perfluoroalcoxy en C₁-C₁₂ ;
R² représente un atome d'hydrogène, un atome de fluor, un atome de chlore, un atome de brome, un groupe alkyle en C₁-C₄, un groupe alcoxy en C₁-C₄, un groupe nitro, un groupe cyano ou un groupe COOR⁴, dans lequel R⁴ représente un atome d'hydrogène ou un groupe alkyle en C₁-C 4 ; et
R³ représente un atome d'hydrogène, un atome de fluor ou un atome de chlore ; ou
deux des radicaux R¹, R² et R³ représentent conjointement un groupe difluorométhylènedioxy, un groupe tétrafluoroéthylène-1,2-dioxy, un groupe 2-oxytétrafluoroéthyle ou un groupe (oxydifluorométhoxy)-difluorométhyle;
**caractérisé en ce que** des composés de formules (IIa) ou (IIb) 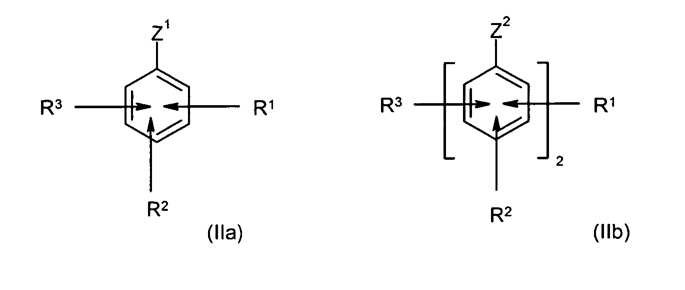 dans lesquelles :
R¹, R² et R³ présentent la signification mentionnée précédemment ;
Z¹ représente un groupe isopropyle, un groupe propényle, un groupe 2-hydroxy-2-propyle, un groupe 2-hydroperoxy-2-propyle ou un groupe 1-méthyloxiranyle ; et
Z² représente un groupe O,O-bis-(2-oxydiisopropyle) ;
sont mis à réagir :
• en présence de 0,1 à 30 % en moles d'un N-hydroxyphtalimide,
• en présence d'un oxydant.

2. Procédé selon la revendication 1, **caractérisé en ce qu'**on utilise des composés de formule (IIa) dans laquelle Z¹ représente un groupe isopropyle et un groupe propényle.

3. Procédé selon au moins l'une des revendications 1 et 2, **caractérisé en ce qu'**on utilise des composés de formule (IIa) dans laquelle Z¹ représente un groupe isopropyle.

4. Procédé selon la revendication 3, **caractérisé en ce que** les composés utilisés sont obtenus par la réaction de composés de formule (III) 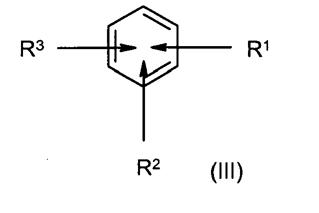 dans laquelle :
R¹, R² et R³ présentent la signification mentionnée dans la revendication 1, avec du propène ou du chlorure d'isopropyle en présence de fluorure d'hydrogène.

5. Procédé selon au moins l'une des revendications 1 à 4, **caractérisé en ce que** la réaction de composés de formules (IIa) et (IIb) donnant lieu à des composés de formule (I) est en outre réalisée :
• en présence de métaux de transition ou de composés de métaux de transition.

6. Procédé selon au moins l'une des revendications 1 à 5, **caractérisé en ce qu'**on utilise des gaz contenant de l'oxygène en tant qu'oxydant.

7. Procédé selon au moins l'une des revendications 1 à 5, **caractérisé en ce que** les réactifs n'ayant pas réagi ou ayant partiellement réagi, ainsi que les métaux de transition ou les composés de métaux de transition éventuellement présents, sont recyclés dans la réaction.
